Europäisches Patentamt

European Patent Office

Office européen des brevets

(11) Publication number:

**0 075 356**

**B1**

(12) **EUROPEAN PATENT SPECIFICATION**

(45) Date of publication of patent specification: **31.07.85**

(51) Int. Cl.⁴: **C 07 C 69/65, C 07 C 67/22**

(21) Application number: **82201115.1**

(22) Date of filing: **09.09.82**

(54) Process for the preparation of 2,2-Dichlorophenylacetic acid esters.

(30) Priority: **17.09.81 NL 8104286**

(43) Date of publication of application:
**30.03.83 Bulletin 83/13**

(45) Publication of the grant of the patent:
**31.07.85 Bulletin 85/31**

(84) Designated Contracting States:
**BE CH DE FR GB IT LI NL**

(56) References cited:
**EP-A-0 006 539**

(73) Proprietor: **STAMICARBON B.V.**
**Postbus 10**
**NL-6160 MC Geleen (NL)**

(72) Inventor: **Corvers, Antonius**
**Kelmonderstraat 33**
**NL-6191 RD Beek (NL)**
Inventor: **Mulders, Joannes M.C.A.**
**V.W. Goudoeverstraat 20**
**NL-6166 XC Geleen (NL)**

(74) Representative: **Hoogstraten, Willem Cornelis**
**Roeland et al**
**OCTROOIBUREAU DSM Postbus 9**
**NL-6160 MA Geleen (NL)**

Courier Press, Leamington Spa, England.

# Description

The invention relates to a process for the preparation of 2,2-dichlorophenylacetic acid esters.

Such compounds can be prepared as known in the art (see Bull. soc. chim. de France 1959 pp. 850—853) by reacting phosphoruspentachloride with phenylglyoxylic acid esters, which esters are prepared from benzoylcyanide.

It has now been found that 2,2-dichlorophenylacetic acid esters can be obtained also from 2,2-dichlorophenylacetonitrile. This nitrile can be obtained as known in the art from benzylcyanide (see J. Chem. Soc. 121, 46, 1922 p. 44), a raw material which is substantially cheaper than benzoylcyanide.

The invention therefore provides a very suitable process for the preparation of 2,2-dichlorophenylacetic acid esters, which process is characterized in that 2,2-dichlorophenylacetonitrile is treated with water in a quantity of more than one mole per mole nitrile and a monovalent alcohol in the presence of a halogenhydracid.

It is to be noted that in the European patent application 6539 a process is described for the preparation of 2-hydroxyphenylacetic acid esters by treating the corresponding nitrile with alcohol, hydrogen chloride and with 0—1 mole water per mole nitrile.

In applying the process according to the invention the desired ester is obtained with a good yield. This is particularly surprising, because, with a view to the presence of water, the formation of the acid corresponding with the ester would be expected. This acid is not formed, however, but depending on the quantity of water, the 2,2-dichlorophenylacetamide is formed to a greater or less extent as byproduct. If no water is used, but only an alcohol and a halogenhydracid, a very small quantity of ester is obtained in addition to a large quantity of the said amide.

The process according to the invention can be performed at different temperatures, for instance temperatures in the range of 0—80°C. Preference is given to applying a temperature ranging from 15 to 50°C.

Preference is given to using hydrogen chloride as halogenhydracid. The chosen quantity of hydrogen chloride may vary, but preferably at least 2 moles hydrogen chloride is used per mole nitrile.

The chosen quantity of alcohol may vary as well, for instance a quantity of 1—50 moles alcohol per mole nitrile. A quantity of more than 50 moles alcohol per mole nitrile can be used also, but this does not result in an advantage. A quantity of 4—30 moles alcohol per mole nitrile is particularly suitable. The choice of the alcohol is determined, of course, by the desired ester. Various monovalent alcohols are suitable for the preparation of the corresponding esters according to the invention, for instance monovalent aliphatic alcohols having from 1—8 carbon atoms, such as e.g. methanol, ethanol, n-propanol, isopropanol, n-butanol, isobutanol, n-pentanol and n-hexanol.

In applying the process according to the invention more than 1 mole water per mole nitrile is used in order to restrict the formation of the amide from the nitrile. A quantity of 2—10 moles water per mole nitrile is particularly suitable. More than 10 moles water per mole nitrile may be used also, but this does not result in an advantage. The amide formed can be recovered from the reaction mixture as byproduct. This amide can be used, for instance, in the preparation of pesticides. The amide formed can be converted also into the ester while applying processes known per se.

The process according to the invention, in the application of which esters can be obtained which are important for the pesticide manufacturing industry, is further elucidated in the following examples.

Example I

A flask having a capacity of 250 ml, provided with baffle plates, stirrer, thermometer and gas inlet tube, is filled with 11.3 g 2,2-dichlorophenylacetonitrile, 47 g methanol and 4.4 g water. The mixture is cooled to 10°C and then saturated with hydrogen chloride gas. During the introduction of the hydrogen chloride gas, the mixture is kept, by cooling, at a temperature of about 20°C. After the introduction of hydrogen chloride gas for about 1 hour, the reaction mixture is allowed to react further at about 25°C for 3 hours more.

The reaction mixture obtained is subsequently poured out into 0.5 l water and the aqueous solution obtained is extracted with ether. The ether extract is neutralized with a sodium-bicarbonate solution to pH=8 and then dried with magnesiumsulphate.

After evaporation of the ether, 10.7 g product remains which, according to a gas-chromatographic analysis, contains 84% by wt. methylester of 2,2-dichlorophenylacetic acid (boiling point 95°C/133 Pa) and 15% by wt. 2,2-dichlorophenylacetamide. The melting range of this amide is 111—112°C.

The yield of ester is 68% of the yield theoretically possible.

Comparative example

In the same way as in example I an experiment is performed without water with 11.3 g 2,2-dichlorophenylacetonitrile and 47 g nonaqueous ethanol as reagents.

After further processing of the reaction mixture, 13.2 g solid matter is obtained. An analysis shows that this product is 2,2-dichlorophenylacetamide having a purity of 85%.

Example II

Example I is repeated, using 47 g ethanol. 75% of the theoretically possible yield of ethylester of 2,2-dichlorophenylacetic acid is obtained. (Boiling point 147°C/2.10$^3$ Pa).

Example III

Example I is repeated, using 47 g ethanol and 8.8 g water. 72% of the theoretically possible yield of ethylester of 2,2-dichlorophenylacetic acid is obtained.

Example IV

In the way described in example I the ethylester is prepared from 30 g 2,2-dichlorophenylacetonitrile, 51 g ethanol and 11.5 g water. The ethylester is obtained with a yield of 73% of the yield theoretically possible.

Example V

Example I is repeated, using 49 g n-butanol instead of methanol. The n-butylester of 2,2-dichlorophenylacetic acid (boiling point 99—100°C/27 Pa) is obtained with a yield of 58% of the yield theoretically possible.

Example VI

Example I is repeated. The hydrogen chloride gas, however, is introduced at a temperature of 40°C. After the introduction of hydrogen chloride gas for about 1 hour, the reaction conditions are maintained at about 40°C for 1 hour more. The yield of ester is 68% of the yield theoretically possible.

**Claims**

1. Process for the preparation of 2,2-dichlorophenylacetic acid esters, characterized in that 2,2-dichlorophenylacetonitrile is treated with water in a quantity of more than one mole per mole nitrile and a monovalent alcohol in the presence of a halogenhydracid.

2. Process according to claim 1, characterized in that per mole nitrile 2—10 moles water are used.

**Patentansprüche**

1. Verfahren zur Herstellung von 2,2-Dichlorphenylessigsäureestern, dadurch gekennzeichnet, daß 2,2-Dichlorphenylacetonitril mit Wasser in einer Menge von mehr als einem Mol je Mol Nitril und einem einwertigen Alkohol in Gegenwart einer Halogenwasserstoffsäure behandelt wird.

2. Verfahren nach Anspruch 1, dadurch gekennzeichnet, daß je Mol Nitril 2—10 Mol Wasser verwendet werden.

**Revendications**

1. Procédé de préparation d'esters de l'acide 2,2-dichlorophénylacétique, caractérisé en ce que le 2,2-dichlorophénylacétonitrile est traité avec de l'eau en quantité supérieure à une mole par mole de nitrile et un alcool monovalent en présence d'un hydracide d'halogène.

2. Procédé selon la revendication 1, caractérisé en ce qu'on utilise par mole de nitrile 2—10 moles d'eau.